Europäisches Patentamt

European Patent Office

Office européen des brevets

(11) Veröffentlichungsnummer: **0 328 848**
**A1**

# (12) EUROPÄISCHE PATENTANMELDUNG

(21) Anmeldenummer: 88810824.8

(22) Anmeldetag: 30.11.88

(51) Int. Cl.⁴: **A61F 2/36**

(30) Priorität: 14.01.88 CH 130/88

(43) Veröffentlichungstag der Anmeldung:
23.08.89 Patentblatt 89/34

(84) Benannte Vertragsstaaten:
AT DE FR GB IT

(71) Anmelder: GEBRÜDER SULZER
AKTIENGESELLSCHAFT
Zürcherstrasse 9
CH-8401 Winterthur(CH)

Anmelder: Protek AG
Stadtbachstrasse 64
CH-3001 Bern(CH)

(72) Erfinder: Morscher, Erwin Walter,
Prof.Dr.-med.
Orthopädische Universitätsklinik Basel
Felix Platter Spital CH-4055 Basel(CH)
Erfinder: Frey, Otto, Dr.
Wallrütistrasse 56
CH-8400 Winterthur(CH)

(54) Netzartige Verstärkung zur Verankerung eines Prothesenschaftes.

(57) Eine netzartige Verstärkung (4), die den Schaft (1) einer Femurkopfprothese (2) mit Abstand umgibt, trennt ein Knochenzementbett (5) in ihrem Inneren von einer Schicht aus verdichtetem spongiosen Gewebe (6) aussen. Sie ist in einzelne Lappen (7) aufgeteilt, die distal an einem Zapfen (8) befestigt sind.

Die sich über die ganze Höhe erstreckenden Schlitze zwischen den Lappen (7) ermöglichen eine weitgehende Aufweitung der netzartigen Verstärkung (4) in radialer Richtung und damit eine Anpassung an den individuellen Operationshohlraum und an Verankerungsschäfte (1) mit unterschiedlich grossen und verschieden geformten Querschnitten.

Fig.1

EP 0 328 848 A1

## Netzartige Verstärkung zur Verankerung eines Prothesen-Schaftes

Die Erfindung betrifft eine netzartige Verstärkung zur Verankerung eines Schaftes einer in einem Röhrenknochen zu verankernden Endoprothese mit Hilfe von Knochenzement, wobei die Verstärkung im Abstand um den Schaft herum angeordnet ist.

Eine derartige Verstärkung ist aus der DE-PS 28 42 847 bekannt. Sie wird in ihrem Inneren mit Knochenzement gefüllt und aussen mit verdichtetem Spongiosa-Gewebe hinterfüllt. Die bekannte Konstruktion besteht dabei aus einem in Umfangsrichtung geschlossenen, sich konisch verjüngenden Träger mit netzartig aufgelöster Wandung. Eine Anpassung dieses Trägers an die Gegebenheiten der Operationshöhlung im individuellen Fall ist in radialer und in Umfangsrichtung relativ schwierig, da Querschnittserweiterungen des Trägerquerschnitts nur im Rahmen der Dehnbarkeit der Stege des Netzes möglich sind.

Aufgabe der Erfindung ist es, die Anpassungsfähigkeit einer netzartigen Verstärkung an die individuellen Gegebenheiten nach allen Richtungen zu gewährleisten.

Diese Aufgabe wird dadurch gelöst, dass die netzartige Verstärkung in über die ganze Höhe geschlitzte Lappen unterreilt ist, die distal an einem im operativ geschaffenen Knochenhohlraum fixierbaren Zapfen befestigt sind.

Mit Hilfe eines Spreitzelementes können die verformbaren Lappen in jeder Richtung an den Operationshohlraum, insbesondere auch an seine Krümmungen, angepasst werden, durch das Aufspreizen wird gleichzeitig die Verdichtung des hinterfüllten Spongiosa-Materials erreicht.

Zweckmässigerweise kann der Zapfen gleichzeitig als Zementsperre dienen. Weiterhin wird mit sich überlappenden Lappen erreicht, dass auch beim Aufweiten der Verstärkung direkte Kontakte des Knochenzementes mit dem Knochengewebe verhindert werden. Um ein stabiles Zementbett im Innern der Verstärkung zu erhalten, ist es weiterhin vorteilhaft, die Aufspreizung des Netzes so weit fortzusetzen, dass bei einer Femurkopfprothese eine Dicke des Zementbettes zwischen Schaft und Netz von mindestens 2 mm eingehalten wird.

Im folgenden wird die Erfindung anhand von Ausführungsbeispielen im Zusammenhang mit der Zeichnung näher erläutert.

Fig. 1 zeigt schematisch in einem Längsschnitt eine mit Hilfe der neuen Verstärkung verankerte Femurkopf-Prothese in einem Femur;

Fig. 2 ist der Schnitt II-II von Fig. 1;

Fig. 3 gibt eine Seitenansicht einer weiteren Ausführungsform der Verstärkung wieder;

Fig. 4 ist der Schnitt IV-IV von Fig. 3.

Ein sogenannter Geradschaft 1 einer Femurkopfprothese 2 ist in einem Femurknochen 3 mit Hilfe einer netzartigen Verstärkung 4 verankert, die ein Knochenzementbett 5 in ihrem Inneren von einer Schicht 6 aus spongiosem Knochengewebe trennt. Diese Schicht 6 wird gebildet aus zuvor operativ aus dem Operationshohlraum gewonnener Spongiosa, mit der nach eventueller Krümelung die Verstärkung 4 aussen hinterfüllt worden ist, wobei die Spongiosa zusätzlich verdichtet wird.

Die Verstärkung 4 besteht aus einzelnen Lappen 7 (Fig. 2 bis 4), deren Trennungsschlitze über die ganze Höhe verlaufen, und aus einer oder mehreren Lagen eines Metalldrahtnetzes oder -gitters aus Titan oder einer Titanlegierung bestehen.

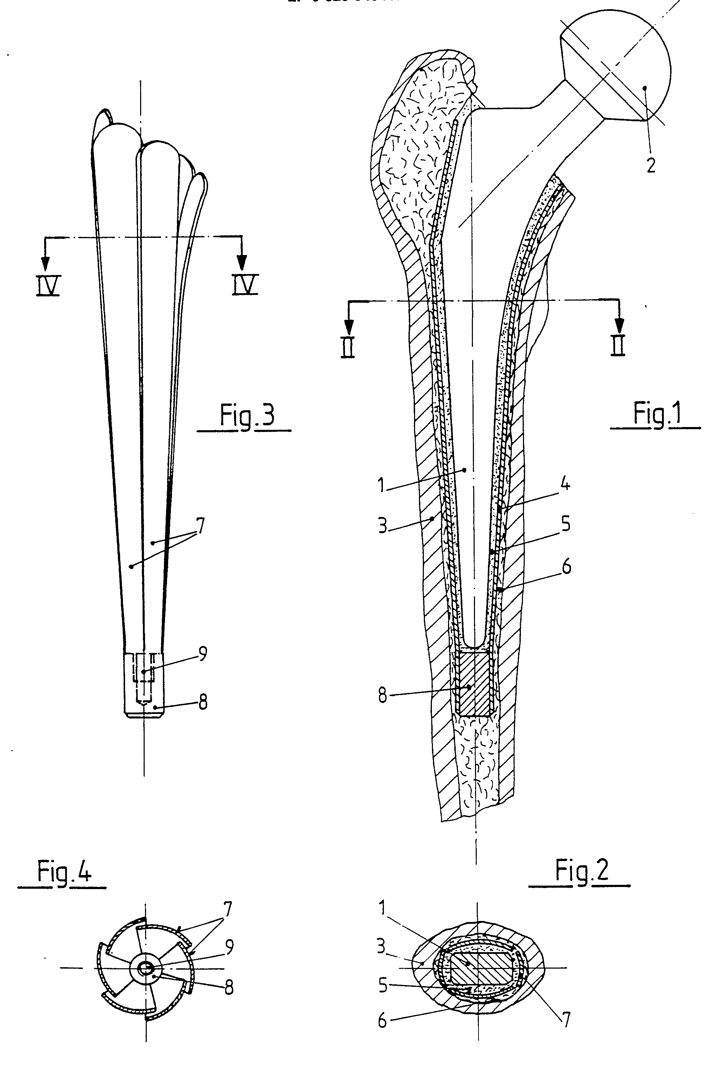

Gehalten sind die Lappen 7 distal an einem Zapfen 8, mit dem sie beispielsweise verschweisst sind. Dieser Zapfen 8 kann gleichzeitig in bekannter Weise als Knochenzementsperre dienen. Er kann weiterhin mit Mitteln, beispielsweise einer Gewindebohrung 9 (Fig. 3 und 4), für die Befestigung eines nicht gezeigten Setzinstrumentes versehen sein.

Um auch beim Aufweiten der netzartigen Verstärkung 4 direkte Kontakte zwischen dem Knochenzementbett 5 und dem verdichteten Gewebe 6 möglichst weitgehend auszuschliessen, können die aus dem Zapfen 8 blütenkelchartig herauswachsenden Lappen 7 sich - sowohl im "Ruhe"- als auch im aufgeweiteten Zustand - in Umfangsrichtung überlappen (Fig. 3 und 4).

Um ein tragfähiges Zementbett 5 zu erhalten, ist es zweckmässig, die Abmessungen der Verstärkung 4 und des Schaftes 1 so aufeinander abzustimmen, dass zwischen beiden ein mindestens 2 mm dickes Zementbett 5 gewährleistet ist.

### Ansprüche

1. Netzartige Verstärkung zur Verankerung eines Schaftes einer in einem Röhrenknochen zu verankernden Endoprothese mit Hilfe von Knochenzement, wobei die Verstärkung im Abstand um den Schaft herum angeordnet ist, **dadurch gekennzeichnet, dass** die netzartige Verstärkung (4) in über die ganze Höhe geschlitzte Lappen (7) unterteilt ist, die distal an einem im operativ geschaffenen Knochenhohlraum fixierbaren Zapfen (8) befestigt sind.

2. Verstärkung nach Anspruch 1, **dadurch gekennzeichnet, dass** der Zapfen (8) als Knochenzementsperre ausgebildet ist.

3. Verstärkung nach Anspruch 1, **dadurch gekennzeichnet, dass** die Lappen (7) sich in Umfangsrichtung überlappen.

4. Verstärkung nach Anspruch 1, **dadurch gekennzeichnet, dass** der Abstand zwischen dem Schaft (1) und der Verstärkung (4) bei einer Femurkopfprothese mindestens 2 mm beträgt.

Fig.3

Fig.1

Fig.4

Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 519 248 (M. TIMOTEO) * Figuren 1,2; Seite 3, Zeilen 7-10; Seite 4, Zeilen 3-9 * --- | 1-3 | A 61 F 2/36 |
| A | GB-A-2 052 267 (HARDINGE et al.) * Figuren 1,3; Zusammenfassung * --- | 1 | |
| A | EP-A-0 220 427 (GEBRÜDER SULZER AG) * Figuren 1,3 * --- | 1 | |
| A | DE-A-2 842 847 (A. VOORHOEVE) * Figuren 2a,4a * ----- | 2,4 | |

RECHERCHIERTE SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 27-04-1989 | ARGENTINI A. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument